# EUROPÄISCHE PATENTSCHRIFT

(11) **EP 1 643 926 B1**
(45) Veröffentlichungstag und Bekanntmachung des Hinweises auf die Patenterteilung: **17.02.2010**
(21) Anmeldenummer: 04740723.4
(22) Anmeldetag: 07.07.2004
(51) Int. Cl.: A61C 13/00, A61C 9/00, G06F 3/033

(54) **VERFAHREN ZUM DARSTELLEN EINES ZAHNTECHNISCHEN OBJEKTES**
METHOD FOR REPRESENTING A DENTAL OBJECT
PROCEDE POUR REPRESENTER UN OBJET DE TECHNIQUE MEDICALE DENTAIRE

(30) Priorität: 14.07.2003 DE 10331989; 12.09.2003 DE 10342537; 05.11.2003 DE 10352217
(43) Veröffentlichungstag der Anmeldung: 12.04.2006
(73) Patentinhaber: DeguDent GmbH, 63457 Hanau-Wolfgang (DE)
(72) Erfinder: VON SCHROETER, Philip, 63517 Rodenbach (DE); ZÖLLNER, Michael, 91257 Pegnitz (DE)
(74) Vertreter: Stoffregen, Hans-Herbert
(86) Internationale Anmeldenummer: PCT/EP2004/007406
(87) Internationale Veröffentlichungsnummer: WO 2005/004742

(56) Entgegenhaltungen:
- EP-A- 1 283 495
- WO-A-98/53428
- DE-A- 19 518 702
- US-B1- 6 287 121

## Beschreibung

Die Erfindung bezieht sich auf ein Verfahren zum Darstellen und Ausrichten eines digitalisierten zahntechnischen Objekts wie Zahnersatz oder Modell zumindest eines Zahnes auf einem Monitor unter Zugrundelegung eines rechtwinkligen Koordinatensystems mit X-, Y- und Z-Achse, wobei die Z-Achse und die Y-Achse und deren Schnittpunkt in der Darstellungsebene des Monitors und die X-Achse senkrecht zu der Darstellungsebene verlaufen.

Standardmäuse wurden zur Navigation in zweidimensionalen Systemen entwickelt, und zwar im Wesentlichen zur Positionierung eines Mauszeigers auf einem Monitor bzw. PC-Bildschirm. Mit solchen Mäusen lassen sich zwei Translationsfreiheilsgrade steuern und über ein zusätzliches Stellrad gegebenenfalls eine weitere Funktion bedienen.

Bei einer vollständigen dreidimensionalen Ausrichtung (Objekt wird bewegt) oder Navigation (Kamera bzw. Betrachter wird bewegt) ist die Ansteuerung von sechs Freiheitsgraden erforderlich, nämlich drei Freiheitsgrade für die Translation und drei für die Rotation. Um dies zu realisieren, erfolgt zumeist eine Kombination von Tastatureingaben und Mausbewegung. Eine intuitive Bedienung ist dabei nicht möglich, vielmehr erfordert es erheblicher Übung und längerer Einarbeitungszeit.

Zur Navigation bzw. Ausrichtung im dreidimensionalen Raum wurden daher verschiedene Eingabegeräte wie Joysticks oder Trackballs (Kugeln) entwickelt. Mit diesen Eingabegeräten können in der Regel alle sechs Freiheitsgrade intuitiv gesteuert werden, gleichwenn eine präzise Navigation bzw. Ausrichtung einer erheblichen Einarbeitung bedarf. Ein Hauptproblem hierbei ist die unerwünschte Überlagerung von zwei oder mehr Bewegungsrichtungen.

Im dentalen Bereich sind keine Systeme bekannt, bei denen eine 3D-Ausrichtung dentaler Modelle mittels eines Eingabegerätes erfolgen kann, das auf die Belange der jeweiligen Aufgabe bzw. der Benutzer abgestimmt ist. Vielmehr werden üblicherveise Standardmäuse benutzt.

Der WO-A 1998/53428 ist ein Verfahren zu entnehmen, um eine orthodontische Diagnose durchführen zu können. Hierzu ist vorgesehen, dass ein Kieferabdruck um die Y-und Z-Achse, die in der Darstellungsebene eines Monitors liegen, schrittweise gedreht wird. Ferner besteht die Möglichkeit eines Zoomens entlang der X-Achse.

Um auf einem Monitor Gegenstände in verschiedenen Positionen darzustellen, sind Eingabeelemente bekannt, wobei zum Beispiel mittels einer SpaceMouse ® ein Zoomen und Drehen bzw. Verschieben des Objektes im erforderlichen Umfang erfolgen kann.

Der US-A- 5,557,714 ist ein Verfahren zu entnehmen, um ein dreidimensionales Modell um zwei senkrecht zueinander verlaufende Achsen drehen zu können.

Aus der US-B-6,287,121 sind ein Verfahren und eine Vorrichtung zur Herstellung eines Zahnersatzteiles bekannt. Dabei besteht die Möglichkeit, den herzustellenden Zahnersatz auf einem Monitor darzustellen.

Eingabetastaturen, um Gegenstände um sechs Freiheitsgrade zu verstellen, sind z. B. aus der EP-A 1 283 495 oder der DE-C 44 05 314 bekannt.

Der vorliegenden Erfindung liegt die Aufgabe zugrunde, ein Verfahren der eingangs genannten Art so weiterzubilden, dass eine intuitive und einfache Ausrichtung virtueller Modelle von zahntechnischen Objekten, insbesondere von Zähnen oder Zahnreihen im

Rahmen der Visualisierung von Scandaten und der CAD-Modellation von Zahnersatz ermöglicht wird. Ferner soll die Möglichkeit geschaffen werden, aufgrund der digitalisierten Daten eines mit dem Zahnersatz zu versehenen Kieferbereichs, wie eine oder mehrere Zahnstümpfe, den virtuellen auf dem Monitor dargestellten Zahnersatz einfach zu überprüfen, um sodann aufgrund dieser Daten den gewünschten Zahnersatz herstellen zu können.

Die Aufgabe wird erfindungsgemäß gelöst durch ein Verfahren zum Darstellen und Ausrichten eines digitalisierten zahntechnischen Objektes wie Zahnersatz oder Modell zumindest eines Zahnes oder eines mit dem Zahnersatz zu versehenen Kieferbereichs auf einem Monitor unter Zugrundelegung eines rechtwinkligen Koordinatensystems mit X-, Y- und Z-Achse, wobei die Z-Achse und die Y-Achse und deren Schnittpunkt in der Darstellungsebene des Monitors und die X-Achse senkrecht zu der Darstellungsebene verlaufen, wobei das zahntechnische Objekt entlang einer in von der X-Achse und von der Y-Achse aufgespannten Ebene verlaufenden und den Ursprung des Koordinatensystems durchsetzenden T-Achse ausgerichtet wird und mit Hilfe von Eingabeelementen eines Eingabegerätes um maximal fünf Freiheitsgrade bewegt wird, wobei jedem Freiheitsgrad ein Eingabeelement des Eingabegerätes zugeordnet ist und als erster Freiheitsgrad eine Rotation um die Z-Achse, als zweiter Freiheitsgrad eine Rotation um die T-Achse, als dritter Freiheitsgrad eine Translation des Objekts entlang der T-Achse und als vierter Freiheitsgrad die Verstellung des Objekts entlang der X-Achse zum Zoomen des Objekts ausgewählt sind.

Im Gegensatz zu technischen 3D-CAD-Systemen, mit deren Hilfe beliebige Objekte darzustellen und zu manipulieren sind - dies erfordert eine Objektbewegung um alle 6 Freiheitsgrade -, erfolgt bei der CAD-Modellation von Zahnersatz erfindungsgemäß eine vereinfachende Reduktion auf 4 bzw. 5 Freiheitsgrade. Es genügt, die virtuellen Zahnkronen und den zu versorgenden Kieferbereich zu betrachten. Umliegende Bereiche wie Kieferknochen, Zunge oder Lippen sind für zahntechnische Restaurationen nicht von Bedeutung und werden auch nicht digitalisiert. Auch die vom Zahnfleisch verdeckte Zahnwurzel und das Innere des Zahnes werden bei der computergestützten Zahnrestauration nicht berücksichtigt.

Mit anderen Worten sieht die Erfindung ein Verfahren zur Ausrichtung eines auf einem Monitor in einem Koordinatensystem dargestellten digitalisierten Ausschnitts eines Objekts, insbesondere einer Zahnreihe, unter Verwendung eines Eingabegerätes vor, wobei das Objekt um maximal fünf, vorzugsweise vier Freiheitsgrade ausgerichtet wird:
1. Rotation um die Z-Achse durch den Koordinatenursprung,
2. Rotation um die Objektlängsachse (T-Achse), die entlang der Zahnreihe durch den Koordinatenursprung verläuft, wobei die Rotation vorzugsweise eingeschränkt ist, also ein Kippen erfolgt,
3. Translation des Objekts entlang der Objektlängsachse (T-Achse) und insoweit eingeschränkt, als dass der Koordinatenursprung stets innerhalb des Objekts liegt, und
4. Translation entlang der X-Achse vom Koordinatenursprung zum Betrachter hin (Zoom).

Der Ursprung des Koordinatensystems sollte im oder im Wesentlichen im Bildschirmmittelpunkt verbleiben. Dabei fällt die Z-Achse mit der Hochachse des zahntechnischen Objekts zusammen, sofern dieses nicht um die T-Achse gedreht ist.

Bei Darstellung größerer Ausschnitte des Zahnbogens kann die Objektlängsachse (T) durch einen Polygonzug gebildet werden, der aus Abschnitten zusammengesetzt ist, die aus geradlinigen Verbindungen zwischen z. B. Kronenmittejpunkten bestehen können.

Kann nach einer Weiterbildung der Erfindung eine Rotation um die X-Achse als fünfter Freiheitsgrad ausgewählt werden, so ist bevorzugterweise jedoch vorgesehen, dass das zahntechnische Objekt maximal entsprechend dem ersten, dem zweiten, dem dritten und dem vierten Freiheitsgrad bewegt wird.

Um eine intuitive und einfache Ausrichtung des dargestellten zahntechnischen Objekts zusätzlich zu erleichtem, sieht eine Weiterbildung der Erfindung vor, dass die Drehung um die T-Achse eingeschränkt wird, d. h., dass im Wesentlichen eine Kippbewegung erfolgt. Dabei kann das Objekt um die T-Achse um einen Winkel α mit α < 360°, insbesondere α ≤ 180° gedreht werden.

Zusätzlich kann eine eingeschränkte Bewegung entlang der T-Achse erfolgen, ohne dass Einbußen in Bezug auf die Darstellung des zahntechnischen Objekts hingenommen werden.

In Weiterbildung der Erfindung ist vorgesehen, dass Längsachse des zahntechnischen Objekts durch einen Polygonzug mit Abschnitten des zahntechnischen Objekts wie Mittelpunkte von Abschnitten des herzustellenden Zahnersatzes wie Kronen verbindenden Geraden gebildet wird, dass zum Verstellen des zahntechnischen Objekts entlang der T-Achse ausschließlich ein Bewegen entlang einer Geraden des Polygonzuges erfolgt, die den Ursprung des Koordinatensystems durchsetzt. Dabei ist zum Verstellen des zahntechnischen Objekts entlang aufeinander folgender einen Winkel β mit β ≠ 180° einschließenden ersten und zweiten Geraden nach Beendigung des Verstellens entlang der ersten Gerade vor Verstellen des zahntechnischen Objekts entlang der zweiten Gerade vorgesehen, dass das zahntechnische Objekt um den Winkel β um die Z-Achse gedreht wird, so dass infolgedessen der Verlauf der zweiten Gerade richtungsmäßig zu vorigem Verlauf der ersten Gerade entspricht.

Das Digitalisieren des mit dem Zahnersatz zu versehenen Kieferbereichs und das Herstellen von Zahnersatz aufgrund digitalisierter Werte, also nach dem CAD-CAM-Verfahren wird z. B. in der WO-A- 99/47065 beschrieben.

Selbstverständlich besteht auch die Möglichkeit, dass nicht nur der Zahnersatz, sondern auch der Kieferbereich dargestellt wird, auf den der Zahnersatz aufzubringen ist, um eine optische Überprüfung auf dem Monitor vornehmen zu können.

In Weiterbildung der Erfindung ist vorgesehen, dass das zum Ausrichten des Objekts verwendete Eingabegerät Eingabeelemente aufweist, über die das Ausrichten des Objekts um die jeweiligen Freiheitsgrade getrennt voneinander durchgeführt wird. Dabei wird als das Eingabegerät insbesondere ein solches mit vier Eingabeelementen verwendet, wobei ein Eingabeelement ein Umschalter zur Doppelbelegung eines weiteren Eingabeelementes sein kann.

In Ausgestaltung ist als ein oder mehrere Eingabeelemente ein Stellrad vorgesehen. Auch kann als Eingabegerät ein die Funktionen von zumindest zwei Eingabeelementen ausübender Trackball verwendet werden.

Nach einer bevorzugten Ausführungsform wird als Eingabegerät ein solches verwendet, das ein erstes und ein zweites jeweils um zumindest eine Achse drehbares Eingabeelement sowie eine Taste als drittes Eingabeelement umfasst, wobei beim Betätigen des ersten Eingabeelementes das Objekt um eine erste Achse (T-Achse) und bei Betätigen des zweiten Eingabeelementes das Objekt um eine senkrecht zur ersten Achse verlaufende zweite Achse (Z-Achse) gedreht wird, bei gleichzeitigem Betätigen des dritten Eingabeelementes und des ersten oder des zweiten Eingabeelementes das Objekt entlang einer der Achsen verschoben wird und bei Betätigen des dritten Eingabeelementes und des zweiten oder ersten Eingabeelementes die Darstellung des Objekts entlang senkrecht zur ersten und zweiten Achse verstellt wird (Zoom).

Bei der Verwendung eines Trackballs (Kugel) als eines der Eingabeelemente kann das Objekt um die erste und zweite Achse sowie um eine senkrecht zu dieser verlaufenden Achse durch analoge Drehung des Trackballs gedreht werden.

Nach einem alternativen Vorschlag ist vorgesehen, dass das Eingabegerät Bedienelemente wie Stellräder aufweist, mit denen jeweils eine der vier Objektbewegungen isoliert ausgeführt werden kann.

Die Bedienelemente sind für eine intuitive Bedienbarkeit in Richtung der auszuführenden Bewegungen angeordnet. Beispielsweise wird die Drehung um die Hochachse durch ein Stellrad mit senkrechter Drehachse angesteuert, die Drehung um die Objektlängsachse und die beiden Translationen durch Stellräder mit waagerechten oder annähernd waagerechten Drehachsen.

Dabei kann zur Verringerung des konstruktiven Aufwands ein Bedienelement für zwei oder drei ähnlich orientierte Bewegungen (z. B. Kippen und Zoom) verwendet werden, wenn es, z. B. mittels eines Umschalters, mehrfach belegt wird.

In einer weiteren Ausführungsform werden die beiden Rotationen durch Verwendung einer Kugel (Trackball) zusammengefasst.

Die Verwendung einer Kugel (Trackball) erlaubt die Einbeziehung der Rotation um die dritte Achse ohne ein zusätzliches Bedienelement. In diesem Fall ist die Objektbewegung auf 5 Freiheitsgrade beschränkt.

Weiterhin ist die Verwendung einer Standardmaus vorgesehen, mit der die vier Bewegungen durch Kombinationen von Mausbewegung, Tastenbetätigung und Betätigung des Stellrades (Scroll) realisiert werden. Allerdings entsprechen hierbei nicht alle Bewegungen von Maus und Bedienelementen den Bewegungen des Objekts.

Insbesondere ist ein Eingabegerät vorgesehen, das die Bewegung des zahntechnischen Objekts um vier Freiheitsgrade ermöglicht. Das Eingabegerät umfasst drei erste Stellräder mit jeweils einer in etwa parallel zu einer Bedienerhand verlaufenden Achse und ein viertes Stellrad mit hierzu in etwa senkrecht verlaufender Achse. Dabei sind zwei erste Stellräder jeweils um eine Achse drehbar, die zusammenfallen oder parallel zueinander verlaufen und das verbleibende erste Stellrad um eine senkrecht zu diesen Achsen verlaufende Achse drehbar. Zwischen den zwei parallel zueinander angeordneten ersten Stellrädern und dem senkrecht hierzu verlaufenden verbleibenden ersten Stellrad ist sodann das vierte Stellrad angeordnet.

Durch eine diesbezügliche Ausbildung des Eingabegerätes ist eine problemlose Einhandbedienung möglich, mit der im gewünschten Umfang das zahntechnische Objekt auf dem Monitor darstellbar und in seiner Ausrichtung zum Beobachter veränderbar ist.

Unabhängig davon, ob das erfindungsgemäße Ausrichtungskonzept anwendungsspezifisch auf vier oder fünf Freiheitsgrade beschränkt ist, erfolgt zusätzlich eine eingeschränkte Bewegung des Objekts, insbesondere in Bezug auf die Translationsbewegung entlang Längsachse des Objektes (T-Achse) sowie gegebenenfalls eingeschränkte Drehung (Kippung) um diese Achse. Eine vollständige Rotation sollte jedoch zumindest um die senkrechte und zur T-Achse orthogonal verlaufende Z-Achse möglich sein.

Erfindungsgemäß und abweichend von technischen CAD-Systemen kann eine Objektausrichtung durch Beschränkung der Freiheitsgrade und durch Trennung der einzelnen Bewegungen vereinfacht werden. Somit kann die erfindungsgemäße Lehre von Personen genutzt werden, die geringe oder nur durchschnittliche PC-Erfahrungen aufweisen. Aufwendige bzw. kostspielige Schulungskurse oder lange Einarbeitungsphasen sind somit nicht erforderlich. Es ist eine einfache intuitive Bedienung möglich.

Weitere Einzelheiten und Vorteile der Erfindung ergeben sich aus der nachfolgenden Beschreibung von der Zeichnung zu entnehmenden bevorzugten Ausfuhrungsbeispielen.

Es zeigen:
- Fig. 1: eine Prinzipdarstellung eines Eingabegerätes mit vier Stellrädern,
- Fig. 1a: eine Ausgestaltung des Eingabegerätes nach Fig. 1,
- Fig. 2: eine Prinzipdarstellung eines Eingabegerätes mit drei Stellrädern und einem Umschalter (Taster),
- Fig.3: eine Prinzipdarstellung eines Eingabegerätes mit einer Kugel (Trackball) und zwei Stellrädern,
- Fig. 4: eine Prinzipdarstellung einer weiteren Ausführungsform eines Eingabegerätes,
- Fig. 5: eine Prinzipdarstellung einer kurzen Zahnreihe zur Erläuterung des erfindungsgemäßen Ausrichtungs- bzw. Navigatorkonzeptes,
- Fig. 6: eine Prinzipdarstellung eines Brückenabschnitts, dessen Längsachse durch einen Polygonzug gebildet wird,
- Fig. 7: Darstellung eines zahntechnischen Objektes in der Ausgangsposition,
- Fig. 8: das zahntechnische Objekt gemäß Fig. 7 nach Drehung um die Z-Achse,
- Fig. 9: das zahntechnische Objekt gemäß Fig. 8 nach Drehung um die T-Achse,
- Fig. 10: das zahntechnische Objekt gemäß Fig. 9 nach Verschiebung entlang der Z-Achse und
- Fig. 11: das zahntechnische Objekt gemäß Fig. 7 nach Verschiebung entlang der T-Achse.

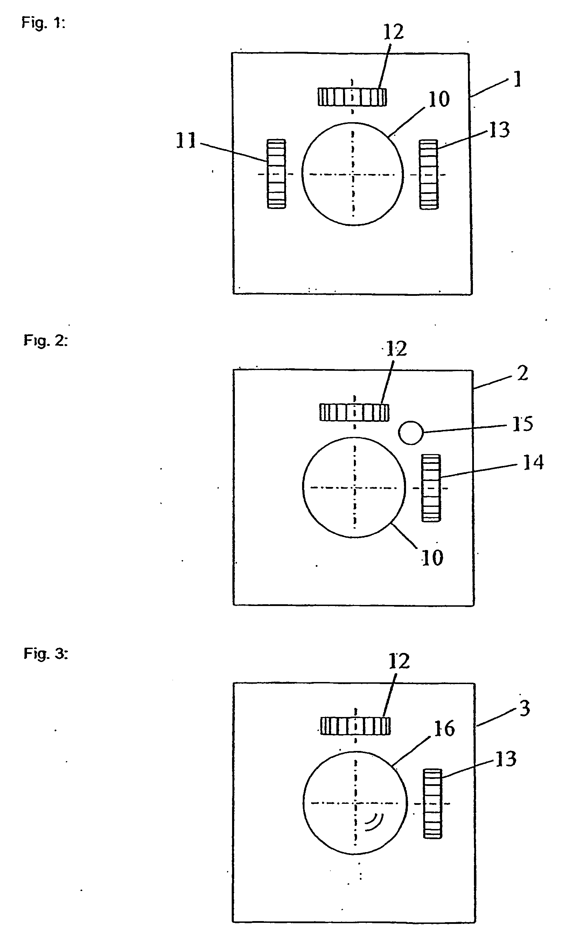

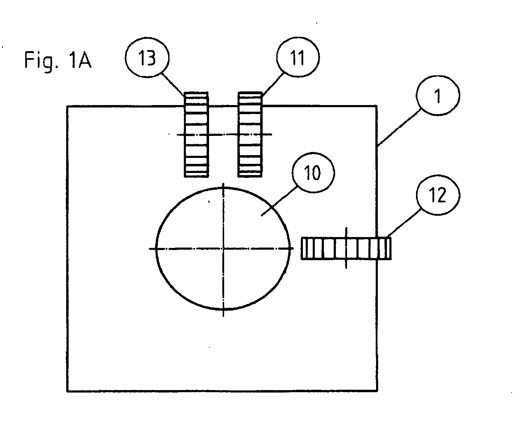

In Fig. 1 bis 3 sind vier Ausführungsformen 1, 2, 3 eines Eingabegerätes dargestellt, um eine Ausrichtung virtueller Modelle von Zahnersatz wie Käppchen 44 bzw. Modell 18, 46 eines Kieferabschnittes im Rahmen der Visualisierung von Scandaten oder der CAD-Modellation von Zahnersatz zu ermöglichen.

Durch die Bedienung einzelner Eingabeelemente 10, 11, 12, 13, 14, 15, 16, 32, 34, 36 bzw. kombinierte Nutzung dieser besteht die Möglichkeit, auf einem Monitor, der an einen PC angeschlossen ist, mit dem wiederum das Eingabegerät 1, 2, 3 verbunden ist, ein Objekt wie das Modell eines Kieferabschnittes bzw. des Zahnersatzes 44 mit anwendungsspezifisch auf vorzugsweise vier, ggfs. fünf Freiheitsgrade eingeschränkter Bewegung navigieren zu können.

Das Modell 18, 46 eines Kieferabschnittes bzw. der Zahnersatz 44 ist in einem rechtwinkligen Koordinatensystem mit X-, Y- und Z-Achse navigierbar. In dem Koordinatensystem verläuft eine mit dem Bezugszeichen 20 versehene T-Achse senkrecht zur Z-Achse 22 und in einer von der X-Achse 24 und der Y-Achse 25 des rechtwinkligen Koordinatensystems aufgespannten Ebene, wobei T-Achse 20 und Y-Achse 25 in Abhängigkeit von der Drehung des Objekts 18, 44, 46 um die Z-Achse 22 zusammenfallen oder einen Winkel zueinander beschreiben können, wie sich dies aus den zeichnerischen Darstellungen ergibt. Unabhängig hiervon gehen die X-Achse 24, die Y-Achse 25 und damit die T-Achse 20 und die Z-Achse 22 von einem Koordinatenursprung 28 aus, der vorzugsweise im Mittelpunkt des Monitors verläuft.

Die T-Achse 20 fällt mit Mittelachse des digitalisierten länglichen Objekts 18, also dem Modellabschnitt, zusammen. Die T-Achse 20 endet an den Objektgrenzen, wird jedoch darüber hinaus angezeigt. Um das Objekt 18 entlang der T-Achse 20 zu verschieben (scheinbare Verschiebung des Koordinatenursprungs 28) (Trans_{T}), wird bei Nutzung aller drei Varianten des Eingabegerätes 1, 2, 3 das Stellrad 12 gedreht. Um eine eingeschränkte Rotation (Kippen) um z. B. ca. 180° um die T-Achse 20 zu ermöglichen (Rot_{T}), wird beim Eingabegerät 1 das Stellrad 11 gedreht und bei Eingabegerät 2 das Stellrad 14. Bei Eingabegerät 3 wird die Kugel (Trackball) 16 um eine horizontale Achse gedreht, die zur Mittelachse (T) des digitalisierten Objektes parallel verläuft.

Um eine vollständige Drehung um die Z-Achse 22 (Rot_{Z}) durchzuführen, wird bei Eingabegeräten 1 und 2 das Stellrad 10 bedient, bei Eingabegerät 3 wird die Kugel 16 um ihre Hochachse (Z) gedreht. Um schließlich ein Zoomen zu ermöglichen, also eine Translation entlang der durch den Koordinatenursprung 28 und senkrecht zu den Achsen 22, 25 verlaufenden Achse 24 durchzuführen, wird bei den Eingabegeräten 1 und 3 das Stellrad 13 benutzt und bei dem Eingabegerät 2 die Taste 15 gedrückt und gehalten und zusätzlich das Stellrad 14 gedreht. Dabei kann der Modellabschnitt 18 nicht "verlorengehen", da der Koordinatenursprung 28 im Mittelpunkt des Monitors verbleibt.

Das Eingabegerät 3 ermöglicht zusätzlich eine Drehung um die zur Hochachse (Z-Achse) und Y-Achse orthogonal verlaufende X-Achse 24. Dazu ist die Kugel 16 um eine horizontale Achse zu drehen, die parallel zur Drehachse der Stellachse 12 verläuft.

Mit den Eingabegeräten 1, 2 oder 3 ist ein Ausrichtungskonzept mit anwendungsspezifisch auf vier Freiheitsgrade eingeschränkter Bewegung möglich, und zwar volle Rotation um die Hoch- bzw. Z-Achse 22, beschränkte Translation (Trans_{T}) entlang der T-Achse 20 und ggf. beschränkte Rotation (Kippen) um die T-Achse 20. Weitere einschränkende und damit vereinfachende Bedingung ist, dass der Koordinatenursprung 28 grundsätzlich im Zentrum des Monitors liegt. Somit kann das darzustellende Objekt 18, 44, 46 beim Zoomen nie außerhalb des Bildschirmausschnitts geraten. Diese auf vier Freiheitsgrade eingeschränkte Bewegung wird erwähntermaßen mit den Eingabegeräten 1, 2 und 3 realisiert. Die volle Rotation um die Z-Achse 22 wird durch Bedienen des Stellrings 10 bzw. der Kugel 16, die beschränkte Translation entlang der T-Achse 20 durch den Stellring 12, die beschränkte Rotation (Kippung) um die T-Achse 20 durch das Stellrad 11 bzw. das Stellrad 14 bzw. durch die Kugel 16 und die Translation entlang der zur Monitorebene senkrecht verlaufenden Ebene (Zoom) durch das Stellrad 13 bzw. 14 bei gehaltener Taste 15 realisiert.

Der Fig. 1a ist eine besonders hervorzuhebende Ausgestaltung eines Eingabegerätes 1 zu entnehmen, das von den Elementen bzw. Anzahl und Funktion dem Eingabegerät 1 gemäß Fig. 1 entspricht, so dass für gleiche Elemente gleiche Bezugszeichen verwendet werden.

So verlaufen die Drehachsen der Stellräder 11, 12 und 13 entlang einer Bedienerhand, wohingegen die Drehachse des Stellrades 10 senkrecht zu dieser verläuft. Ferner sind zwei Stellräder - im Ausfühnrugsbeispiel die Stellräder 11 und 13 - parallel zueinander und nebeneinander angeordnet und sind folglich quasi um eine gemeinsame Achse bzw. um parallel zueinander verlaufende Achsen drehbar. Das ebenfalls eine entlang der Bedienerhand verlaufende Drehachse aufweisende Stellrad 12 verläuft in Bezug auf seine Achse senkrecht zu denen der Stellräder 11 und 13, so dass diese Achsen insgesamt in etwa einen rechten Winkel einschließen. Zwischen den nebeneinander angeordneten Stellrädern 11 und 13 und dem mit seiner Achse um 90° zu diesem gedrehten Stellrad 12 ist das Stellrad 10 angeordnet. Dabei verläuft dessen Achse im Schnittpunkt einer von dem Stellrad 12 aufgespannten Mittelebene und einer zwischen den Stellrädern 11 und 13 verlaufenden Ebene, so dass sich ein gutes Erfassen der Stellräder 10, 11, 12, 13 mit einer einzigen Bedienerhand, vorzugsweise der linken, ergibt. Mit dem Stellrad 12 kann eine Bewegung eines zahntechnischen Objekts entlang der T-Achse vorgenommen werden. Hierzu wird vorzugsweise der Daumen verwendet. Mit dem Stellrad 11, das mit einem Zeigefinger bedient werden kann, kann eine Drehung um die T-Achse erfolgen. Mit dem mittleren Stellrad 10, das wiederum mit dem Daumen bewegt werden kann, erfolgt eine Drehung um die Z-Achse. Schließlich wird vorzugsweise mit einem Mittelfinger das Stellrad 13 betätigt, um ein Verschieben des Objekts entlang der X-Achse, also ein gewünschtes Zoomen vornehmen zu können.

Soll ein Ausrichtungskonzept mit anwendungsspezifisch auf fünf Freiheitsgrade eingeschränkter Bewegung erfolgen, so kann ein Eingabegerät benutzt werden, das einen Trackball (Kugel) sowie zwei Stellringe umfasst. Durch Nutzung der entsprechenden Eingabeelemente einzeln oder in Kombination besteht die Möglichkeit, eine Rotation um die T-Achse, eine Rotation um die Z-Achse, eine Rotation um die senkrecht zu der Monitorebene und der Z-Achse verlaufende X-Achse, eine beschränkte Translation entlang der T-Achse, also entlang Längserstreckung des zu navigierenden Objektes sowie eine Translation entlang der zur Monitorebene orthogonal verlaufenden X-Achse (Zoom) durchzuführen. Dies kann aufgrund folgender Eingabeelementennutzung erfolgen:
- die Rotation um die horizontale Achse (T-Achse) durch analoge Drehung des Trackballs,
- die Rotation um die vertikale Achse (Z-Achse) durch analoge Drehung des Trackballs,
- die Rotation um die senkrecht zu der horizontal und vertikal verlaufenden Achse durch analoge Drehung des Trackballs,
- die beschränkte Translation entlang der Längserstreckung des Objektes, also der T-Achse durch den ersten Stellring, und
- das Zoomen (Translation) entlang der senkrecht zur Monitorebene verlaufenden Achse durch den zweiten Stellring.

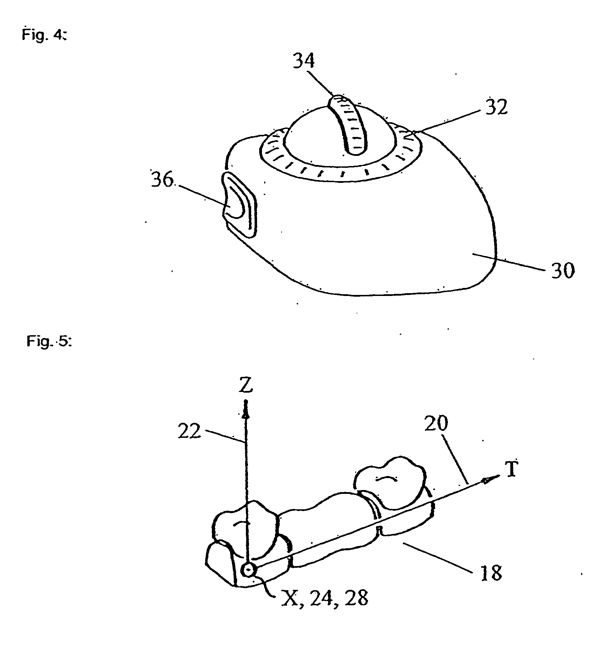

In der Fig. 4 ist eine weitere Ausführungsform eines Eingabegerätes 30 dargestellt, um eine Navigation virtueller Modelle von Zähnen oder Zahnreihen im Rahmen der Visualisierung von Scandaten oder der CAD-Modellation von Zahnersatz zu ermöglichen. Die Maus 30 umfasst eine Drehscheibe bzw. einen Stellring ("JOG DIAL") 32 als erstes Bedienelement und ein von diesem umgebenes Drehrad ("Scroll") 34 oder einen Trackball als zweites Bedienelement sowie eine Taste 36 als drittes Bedienelement.

Durch die Bedienung der einzelnen Eingabeelemente bzw. kombinierte Nutzung dieser besteht die Möglichkeit, auf einem Monitor, der an einen PC angeschlossen ist, mit dem wiederum die Maus verbunden ist, ein Objekt wie den Modellabschnitt 18, 46 anwendungsspezifisch auf vier bzw. fünf Freiheitsgrade eingeschränkter Bewegung navigieren zu können.

Um das Objekt 18, 44, 46 entlang der T-Achse 20 zu verschieben (scheinbare Verschiebung des Koordinatenursprungs) (Trans_{T}) wird bei Nutzung des Eingabegerätes 30 die Taste 36 zusammen mit der Drehscheibe bzw. dem Stellring 32 benutzt. Um eine eingeschränkte Rotation (Kippen) um ca. 180° um die T-Achse 20 zu ermöglichen (Rot_{T}), wird die Drehscheibe bzw. der Stellring 34 benutzt.

Um eine vollständige Drehung um die Z-Achse 22 (Rot_{Z}) durchzuführen, wird das Dreh- bzw. Stellrad 34 bedient. Um schließlich ein Zoomen zu ermöglichen, also Translation entlang der durch den Koordinatenursprung 28 und senkrecht zu der Achse 22 verlaufenden Achse 24 (X-Achse) durchzuführen, werden die Taste 36 und das Dreh- bzw. Stellrad 34 gleichzeitig benutzt. Dabei kann das Objekt 18 nicht "verlorengehen", da der Koordinatenursprung 28 im Mittelpunkt des Monitors verbleibt.

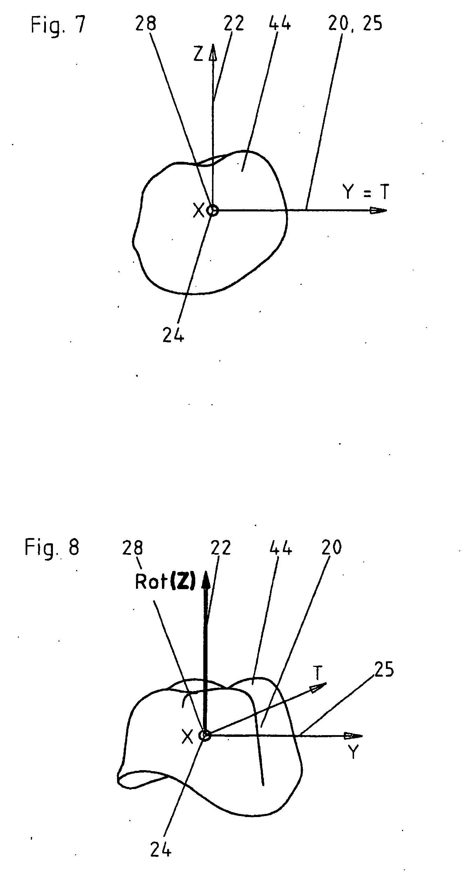

Die erfindungsgemäße Lehre zum Navigieren eines zahntechnischen Objekts auf einem Monitor, wobei das zahntechnische Objekt maximal um fünf, bevorzugtenveise um vier Freiheitsgrade bewegbar ist, soll anhand der Fig. 7 bis 11 näher erläutert werden. Als zahntechnisches Objekt wird dabei ein Käppchen 44 betrachtet.

In Fig. 7 befindet sich das Käppchen 44 in der Ausgangssituation, d. h. dass der Ursprung 28 des Koordinatensystems in etwa mittig in dem Käppchen 44 verläuft. Die mit der Hochachse des Käppchens 44 zusammenfallende Z-Achse ist - wie zuvor - mit dem Bezugszeichen 22 und die Y-Achse mit dem Bezugszeichen 25 gekennzeichnet. Senkrecht zu der Y-Achse 25 und der Z-Achse 22, also zu der von der Darstellungsebene des Monitors aufgespannten Ebene verläuft die den Ursprung 28 durchsetzende X-Achse 24. Die Y-Achse 25 fällt in der Ausgangsstellung des Käppchens 44 mit der T-Achse 20 zusammen, die sich entlang Längsachse des Käppchens 44 erstreckt. Wird das Käppchen 44 um die Z-Achse 22 als ersten Freiheitsgrad (Rot(Z)) gedreht, so bewegt sich entsprechend die T-Achse 20 mit, wie sich aus der Darstellung der Fig. 8 ergibt.

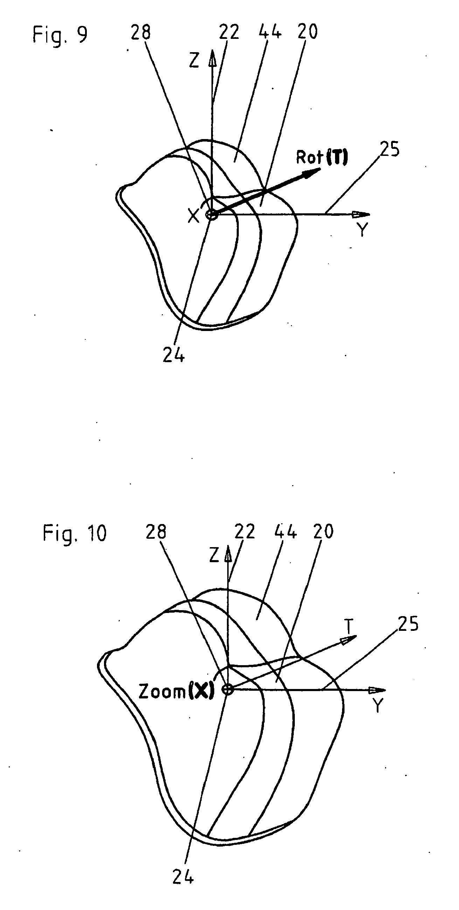

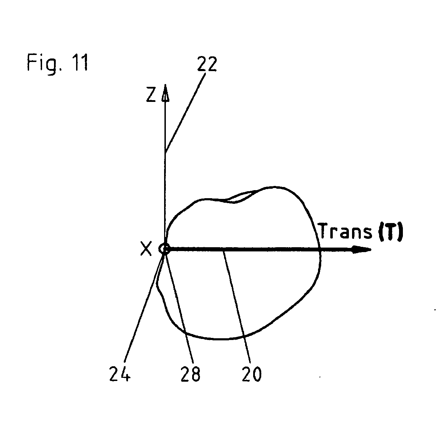

In Fig. 9 erfolgt ein Kippen (Rot(T)) um die T-Achse 20 als zweiter Freiheitsgrad. Ein Verstellen des Käppchens 44 entlang der T-Achse 20 als dritter Freiheitsgrad wird durch die Fig. 11 verdeutlicht. Dabei verläuft die T-Achse 20 in der Darstellungsebene, fällt folglich mit der Y-Achse 25 zusammen. Dies ist jedoch kein zwingendes Merkmal. Schließlich wird als vierter Freiheitsgrad ein Verstellen des Käppchens 44 entlang der X-Achse in Fig. 10 verdeutlicht, ohne dass die Y-Achse 25 und die T-Achse 20 zusammenfallen müssen.

Die Fig. 11 soll des Weiteren vermitteln, dass die Bewegung des Käppchens 44 entlang der T-Achse 20 eingeschränkt dahingehend erfolgt, dass das Käppchen 44 grundsätzlich nur derart zum Ursprung 28 positionierbar ist, dass dieser das Käppchen 44 bzw. ein ansonsten dargestelltes zahntechnisches Objekt schneidet.

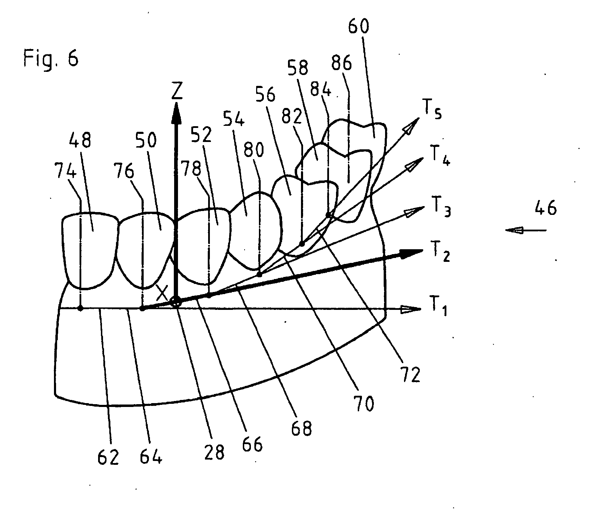

In den Fig. 5 und 7 bis 11 ist ein mehrgliedriger Modellabschnitt 18 bzw. ein Käppchen 44 dargestellt, dessen jeweilige Längsachse entlang einer Geraden verläuft, entlang der die T-Achse 20 ausgerichtet wird. Die erfindungsgemäße Lehre ist jedoch auch für zahntechnische Objekte realisierbar, bei denen der Zahnersatz bzw. der Modellabschnitt, d. h. dessen Längsachse entlang eines Bogens verläuft, wie dies der Fig. 6 zu entnehmen ist. In dieser ist ein mehrgliedriger Modellabschnitt 46 dargestellt, der aus einzelnen Gliedern 48, 50, 52, 54, 56, 58, 60 besteht. Ein Polygonzug 62 wird dem Bogen angepasst, auf dem die Glieder 48, 50, 52, 54, 56, 58, 60 angeordnet sind. Der Polygonzug 62 setzt sich seinerseits aus Geraden 64, 66, 68, 70, 72 zusammen, wie dies anhand der Fig. 6 verdeutlicht wird. Dabei verlaufen die Geraden 64, 66, 68, 70, 72 zwischen Mittelpunkten 74, 76, 78, 80, 82, 84, 86 der Glieder 48, 50, 52, 54, 56, 58, 60 des mehrgliedrigen Modellabschnitts 46.

Um den mehrgliedrigen Modellabschnitt 46 um die T-Achse als zweiten Freiheitsgrad zu drehen bzw. zu kippen oder entlang der T-Achse als dritten Freiheitsgrad zu verschieben, muss stets eine Gerade des Polygonzuges 62 den Ursprung 28 des Koordinatensystems durchsetzen, wobei die entsprechende Gerade - im Ausführungsbeispiel die Gerade 66 - die momentan wirksame T-Achse (im Ausführungsbeispiel T₂-Achse) vorgibt, entlang der der mehrgliedrige Modellabschnitt 46 verschoben bzw. um welche dieser gedreht bzw. gekippt werden kann. Beim Verschieben des Modellabschnitts 46 entlang des Polygonzuges muss dabei stets eine Gerade 62, 64, 66, 68, 70, 72 den Ursprung 28 durchsetzen. Beim Übergang von einer Geraden auf eine andere Gerade ist es dabei nicht erforderlich, dass der Modellabschnitt 46 gedreht wird. Vielmehr verändert sich entsprechend dem Verlaufs des Polygonzugs 62 die jeweilige momentan wirksame T-Achse, die den zweiten und dritten Freiheitsgrad vorgibt. Mit anderen Worten ist eine Ausrichtung der jeweiligen momentan wirksamen T-Achse auf eine vorherige bzw. nachfolgende nicht erforderlich.

Alternativ besteht die Möglichkeit, beim Verschieben des Modellabschnitts 46 entlang des die scheinbare Längsachse vorgebenden Polygonzugs 62 die den Ursprung 28 momentan durchsetzende T-Achse derart auszurichten, dass jede momentan den Ursprung 28 durchsetzende T-Achse den gleichen Winkel zur X- bzw. Y-Achse des Koordinatensystems einschließt. Wird folglich der Modellabschnitt 46 zunächst entlang der Geraden 66 verschoben, so wird dann, wenn der die Geraden 64 bzw. 68 begrenzende Knickpunkt 88 bzw. 90 erreicht wird, der Modellabschnitt 46 derart um die Z-Achse 22 gedreht, dass die durch die Gerade 64 bzw. 68 vorgegebene Achse T₁ bzw. T₃ mit der vorhergehenden Achse T₂ zusammenfällt.

Mit anderen Worten wird der Modellabschnitt 46 um die Z-Achse 22 um einen Winkel gedreht, der dem Winkel entspricht, den die Geraden 64, 66 bzw. 66, 68 einschließen. Entsprechend wird sukzessiv der Modellabschnitt zu dem Ursprung 28 verstellt und gedreht.

Aufgrund der erfindungsgemäßen Lehre wird eine intuitive und einfache Ausrichtung virtueller Modelle bzw. virtuellen Zahnersatz im Rahmen der Visualisierung von Scandaten und der CAD-Modellation von Zahnersatz ermöglicht.

## Patentansprüche

1. Verfahren zum Darstellen und Ausrichten eines digitalisierten zahntechnischen Objektes wie Zahnersatz oder Modell zumindest eines Zahnes oder eines mit dem Zahnersatz zu versehenen Kieferbereichs auf einem Monitor unter Zugrundelegung eines rechtwinkligen Koordinatensystems mit X-, Y- und Z-Achse, wobei die Z-Achse und die Y-Achse und deren Schnittpunkt in der Darstellungsebene des Monitors und die X-Achse senkrecht zu der Darstellungsebene verlaufen, **dadurch gekennzeichnet, dass**
das zahntechnische Objekt entlang einer in von der X-Achse und von der Y-Achse aufgespannten Ebene verlaufenden und den Ursprung des Koordinatensystems durchsetzenden T-Achse ausgerichtet wird und
mit Hilfe von Eingabeelementen eines Eingabegerätes um maximal fünf Freiheitsgrade bewegt wird, wobei
jedem Freiheitsgrad ein Eingabeelement des Eingabegerätes zugeordnet ist und als erster Freiheitsgrad eine Rotation um die Z-Achse, als zweiter Freiheitsgrad eine Rotation um die T-Achse, als dritter Freiheitsgrad eine Translation des Objekts entlang der T-Achse und als vierter Freiheitsgrad die Verstellung des Objekts entlang der X-Achse zum Zoomen des Objekts ausgewählt sind.

2. Verfahren nach Anspruch 1,
**dadurch gekennzeichnet,**
**dass** das zahntechnische Objekt maximal um den ersten, zweiten, dritten und den vierten Freiheitsgrad bewegt wird.

3. Verfahren nach Anspruch 1,
**dadurch gekennzeichnet,**
**dass** als fünfter Freiheitsgrad eine Rotation (Rot_{X}) des Objekts um die X-Achse gewählt wird.

4. Verfahren nach Anspruch 1,
**dadurch gekennzeichnet,**
**dass** das zahntechnische Objekt maximal um einen Winkel α um die T-Achse gedreht wird, wobei α < 360°, insbesondere α ≤ 180° gewählt wird.

5. Verfahren nach Anspruch 1,
**dadurch gekennzeichnet,**
**dass** das zahntechnische Objekt derart auf dem Monitor dargestellt wird, dass das zahntechnische Objekt unabhängig von dessen Bewegung oder Darstellung von dem Ursprung des Koordinatensystems durchsetzt wird, wobei eine eingeschränkte Translation des Objekts entlang der T-Achse erfolgt.

6. Verfahren nach Anspruch 1,
**dadurch gekennzeichnet,**
**dass** die Längsachse des zahntechnischen Objekts durch einen Polygonzug mit Abschnitte des zahntechnischen Objekts verbindenden Geraden gebildet wird,
**dass** zum Verstellen des zahntechnischen Objektes entlang der T-Achse das zahntechnische Objekt entlang jener Geraden des Polygonzuges verstellt wird, die den Ursprung des Koordinatensystems durchsetzt.

7. Verfahren nach Anspruch 6,
**dadurch gekennzeichnet,**
**dass** zum Verstellen des zahntechnischen Objekts entlang aufeinander folgender einen Winkel β mit β ≠ 180° einschließenden ersten und zweiten Geraden nach Beendigung des Verstellens entlang der ersten Gerade vor Verstellen des zahntechnischen Objekts entlang der zweiten Gerade das zahntechnische Objekt um den Winkel β um die Z-Achse gedreht wird.

8. Verfahren nach zumindest Anspruch 1,
**dadurch gekennzeichnet,**
**dass** eine eingeschränkte Translation des Objekts entlang der T-Achse erfolgt.

9. Verfahren nach zumindest Anspruch 1,
**dadurch gekennzeichnet,**
**dass** das Koordinatensystem mit seinem Ursprung derart auf dem Monitor festgelegt wird, dass der Ursprung unabhängig von der Bewegung des Objekts in vorgegebener Position auf dem Monitor verbleibt.

10. Verfahren nach zumindest Anspruch 9,
**dadurch gekennzeichnet,**
**dass** der Koordinatenursprung in oder etwa in Mittelpunkt des Monitors gelegt wird.

11. Verfahren nach zumindest Anspruch 1 ;
**dadurch gekennzeichnet,**
**dass** die Rotation um die T-Achse durch Hin- und Herschwenken des Objekts als beschränkte Rotation realisiert wird.

12. Verfahren nach zumindest Anspruch 1,
**dadurch gekennzeichnet,**
**dass** zum Ausrichten des Objekts auf dem Monitor ein Eingabegerät verwendet wird, das Eingabeelemente aufweist, über die das Ausrichten des Objekts um die jeweiligen Freiheitsgrade getrennt voneinander durchgeführt wird.

13. Verfahren nach Anspruch 12,
**dadurch gekennzeichnet,**
**dass** als das Eingabegerät ein solches mit vier Eingabeelementen verwendet wird.

14. Verfahren nach zumindest Anspruch 12,
**dadurch gekennzeichnet,**
**dass** als ein Eingabeelement ein Umschalter zur Doppelbelegung eines weiteren Eingabeelementes verwendet wird.

15. Verfahren nach zumindest Anspruch 12,
**dadurch gekennzeichnet,**
**dass** als ein oder mehrere Eingabeelemente ein Stellrad verwendet wird.

16. Verfahren nach zumindest Anspruch 12,
**dadurch gekennzeichnet,**
**dass** als das Eingabegerät ein Funktionen von zumindest zwei Eingabeelemenien ausübender Trackball verwendet wird.

17. Verfahren nach Anspruch 16,
**dadurch gekennzeichnet,**
**dass** bei Verwendung eines Trackballs (Kugel) als eines der Eingabeelemente das Objekt um die erste und zweite Achse sowie um eine senkrecht zu dieser verbaufende Achse durch analoge Drehung des Trackballs gedreht wird.

18. Verfahren nach zumindest Anspruch 12,
**dadurch gekennzeichnet,**
**dass** das Objekt durch wahlweises Betätigen einzelner Eingabeelemente sowie kombiniertes Betätigen von zwei Eingabeelementen auf vier Freiheitsgrade eingeschränkt bewegt wird.

19. Verfahren nach zumindest Anspruch 1,
**dadurch gekennzeichnet,**
**dass** die T-Achse derart ausgerichtet wird, dass diese mit der y-Achse zusammenfällt.

## Claims

1. Method for representing and aligning a digitized dental technical object, such as prosthesis or model of at least a tooth or a jaw area to be provided with the prosthesis, on a monitor, on the basis of a right-angled coordinate system with X-, Y- and Z-axis, whereby the Z-axis and the Y-axis and their intersection in the image plane of the monitor and the X-axis run perpendicular to the image plane
**characterized in**
**that** the dental technical object is aligned along a T-axis running in a plane defined by the X-axis and the Y-axis and passing through the origin of the coordinate system, and is moved to a maximum of five degrees of freedom by means of input elements of an input device, whereby an input element of the input device is assigned to each degree of freedom and a rotation about the Z-axis is chosen as the first degree of freedom, a rotation about the T-axis is chosen as the second degree of freedom, a translation of the object along the T-axis is chosen as the third degree of freedom and the shifting of the object along the X-axis for zooming the object is chosen as the fourth degree of freedom.

2. Method according to claim 1,
**characterized in**
**that** the dental technical object is moved to a maximum of the first, second, third and fourth degree of freedom.

3. Method according to claim 1,
**characterized in**
**that** as fifth degree of freedom, a rotation (Rotₓ) of the object about the X-axis is chosen.

4. Method according to claim 1,
**characterized in**
**that** the dental technical object is maximally rotated at an angle α about the T-axis, whereby α <360°, particularly α ≤ 180° is selected.

5. Method according to claim 1,
**characterized in**
**that** the dental technical object is displayed on the monitor in such a way that the dental technical object is passed through by the origin of the coordinate system independent of its movement or display, with the object being restrictively translated along the T-axis.

6. Method according to claim 1,
**characterized in**
**that** the longitudinal axis of the dental technical object is formed through a traverse with straight lines connecting sections of the dental technical object, that for shifting of the dental technical object along the T-axis the dental technical object is shifted along that straight line of the traverse, which passes through the origin of the coordinate system.

7. Method according to claim 6,
**characterized in**
**that** for shifting the dental technical object along successive first and second straight lines forming an angle β with β ≠ 180°, after completing the shifting along the first straight line before shifting the dental technical object along the second straight line, the technical dental object is rotated at an angle β about the Z-axis.

8. Method according to at least claim 1,
**characterized in**
**that** a restricted translation of the object along the T-axis results.

9. Method according to at least claim 1,
**characterized in**
**that** the coordinate system with its origin is specified on the monitor in such a way that the origin remains in defined position on the monitor independent of the movement of the object.

10. Method according to at least claim 9,
**characterized in**
**that** the coordinate origin is placed in or approximately in the center of the monitor.

11. Method according to at least claim 1,
**characterized in**
**that** the rotation about the T-axis by pivoting the object to and fro is realized as restricted rotation.

12. Method according to at least claim 1,
**characterized in**
**that** for aligning the object on the monitor an input device is used, via which the alignment of the object is arranged at the respective degrees of freedom separately from each other.

13. Method according to claim 12,
**characterized in**
**that** as the input device is used such a one with four input elements.

14. Method according to at least claim 12,
**characterized in**
**that** as an input element a changeover switch is used for double-configuration of a further input element.

15. Method according to at least claim 12,
**characterized in**
**that** as one or several input elements an adjusting wheel is used.

16. Method according to at lest claim 12,
**characterized in**
**that** as the input device is used a track ball carrying out the functions of at least two input elements.

17. Method according to claim 16,
**characterized in**
**that** when using a track ball (ball) as one of the input elements, the object is rotated about the first and second axis, as well as about an axis that runs perpendicular to this by analogous rotation of the track ball.

18. Method according to at least claim 12,
**characterized in**
**that** the object is moved restrictedly to four degrees of freedom by optional actuation of single input elements, as well as by combined actuation of two input elements.

19. Method according to at least claim 1,
**characterized in**
**that** the T-axis is aligned such that it coincides with the Y-axis.

## Revendications

1. Procédé pour représenter et orienter sur un écran un objet de technique dentaire numérisé, tel qu'une prothèse dentaire ou le modèle d'au moins une dent ou d'une partie de la mâchoire destinée à recevoir la prothèse dentaire, sur la base d'un système de coordonnées rectangle avec un axe X, Y et Z, sachant que l'axe Z et l'axe Y et leur point d'intersection s'étendent sur le plan de représentation de l'écran et l'axe Y perpendiculairement au plan de représentation,
**caractérisé en ce**
**que** l'objet de technique dentaire est orienté le long d'un axe T s'étendant sur un plan formé par l'axe X et l'axe Y et traversant l'origine du système de coordonnées, et est déplacé au maximum de cinq degrés de liberté à l'aide d'éléments d'entrée d'un appareil de saisie, sachant qu'un élément d'entrée de l'appareil de saisie est attribué à chaque degré de liberté et qu'est choisi comme premier de degré de liberté une rotation autour de l'axe Z, comme deuxième degré de liberté une rotation autour de l'axe T, comme troisième degré de liberté une translation de l'objet le long de l'axe T, et comme quatrième degré de liberté le déplacement de l'objet le long de l'axe X pour zoomer l'objet.

2. Procédé selon la revendication 1,
**caractérisé en ce**
**que** l'objet de technique dentaire est déplacé au maximum du premier, deuxième, troisième et quatrième degré de liberté.

3. Procédé selon la revendication 1,
**caractérisé en ce**
**qu'**est choisie comme cinquième degré de liberté une rotation (Rot_{X}) de l'objet autour de l'axe X.

4. Procédé selon la revendication 1,
**caractérisé en ce**
**que** l'objet de technique dentaire est tourné au maximum d'un angle α autour de l'axe T, sachant que α < 360°, en particulier α ≤ 180°.

5. Procédé selon la revendication 1,
**caractérisé en ce**
**que** l'objet de technique dentaire est représenté de telle manière sur l'écran que l'objet de technique dentaire est traversé par l'origine du système de coordonnées indépendamment de son mouvement ou de sa représentation, sachant qu'a lieu une translation limitée de l'objet le long de l'axe T.

6. Procédé selon la revendication 1,
**caractérisé en ce**
**que** l'axe longitudinal de l'objet de technique dentaire est formé par un cheminement polygonal avec des droites reliant des sections de l'objet de technique dentaire, et que, pour être déplacé le long de l'axe T, l'objet de technique dentaire est déplacé le long de chaque droite du cheminement polygonal qui traverse l'origine du système de coordonnées.

7. Procédé selon la revendication 6,
**caractérisé en ce**
**que,** pour déplacer l'objet de technique dentaire le long des première et deuxième droites se succédant et comprenant un angle β de β ≠ 180°, l'objet de technique dentaire est tourné de l'angle β autour de l'axe Z après la fin du déplacement le long de la première droite et avant le déplacement de l'objet de technique dentaire le long de la deuxième droite.

8. Procédé selon au moins la revendication 1,
**caractérisé en ce**
**qu'**a lieu une translation limitée de l'objet le long de l'axe T.

9. Procédé selon au moins la revendication 1,
**caractérisé en ce**
**que** le système de coordonnées et son origine sont déterminés de telle façon sur l'écran que l'origine reste dans la position prédéterminée sur l'écran, indépendamment du mouvement de l'objet.

10. Procédé selon au moins la revendication 9,
**caractérisé en ce**
**que** l'origine des coordonnées est située sur ou approximativement sur le point central de l'écran.

11. Procédé selon au moins la revendication 1,
**caractérisé en ce**
**que** la rotation autour de l'axe T est réalisée par pivotement de l'objet dans un sens et dans l'autre sous forme de rotation limitée.

12. Procédé selon au moins la revendication 1,
**caractérisé en ce**
**que**, pour orienter l'objet sur l'écran, est utilisé un appareil de saisie qui présente des éléments d'entrée par l'intermédiaire desquels l'objet est orienté des différents degrés de liberté, séparément les uns des autres.

13. Procédé selon la revendication 12,
**caractérisé en ce**
**qu'**un appareil de saisie à quatre éléments d'entrée est utilisé comme appareil de saisie.

14. Procédé selon au moins la revendication 12,
**caractérisé en ce**
**qu'**un commutateur est utilisé comme élément d'entrée pour la double attribution d'un autre élément d'entrée.

15. Procédé selon au moins la revendication 12,
**caractérisé en ce**
**qu'**une roulette de réglage est utilisée en tant qu'un ou plusieurs éléments d'entrée.

16. Procédé selon au moins la revendication 12,
**caractérisé en ce**
**qu'**une boule de commande exerçant les fonctions d'au moins deux éléments d'entrée est utilisée comme appareil de saisie.

17. Procédé selon au moins la revendication 16,
**caractérisé en ce**
**que**, si une boule de commande (trackball) est utilisée comme un des éléments d'entrée, l'objet est tourné autour du premier et du deuxième axe ainsi qu'autour d'un axe s'étendant perpendiculairement à celui-ci par une rotation analogue de la boule de commande.

18. Procédé selon au moins la revendication 12,
**caractérisé en ce**
**que** l'objet est déplacé de manière limitée à quatre degrés de liberté par l'actionnement choisi de différents éléments d'entrée ainsi que par l'actionnement combiné de deux éléments d'entrée.

19. Procédé selon au moins la revendication 1,
**caractérisé en ce**
**que** l'axe T est orienté de telle façon qu'il coïncide avec l'axe Y.
